# EUROPEAN PATENT APPLICATION

(11) **EP 3 108 807 A1**
(43) Date of publication of application: **28.12.2016**
(21) Application number: 16176051.7
(22) Date of filing: 23.06.2016
(51) Int. Cl.: A61B 5/00

(54) **BONE HEALING PROBE**

(30) Priority: 26.06.2015 US 201562185080 P
(71) Applicant: Stryker European Holdings I, LLC, Kalamazoo, MI 49002 (US)
(72) Inventor: BUESCHER, Robin, 24226 Heikendorf (DE); NASSUTT, Roman, 24143 Kiel (DE)
(74) Representative: Regimbeau

(57) **Abstract**

A bone healing monitoring system (10), which includes a probe configured to measure at least one physical condition. The system includes an optical fiber (20) and a control unit (30) attached to the optical fiber (10). The system (10) also includes a surgically implantable device (40, 140, 240, 340) having a channel extending along at least a portion of the implantable device and is configured to receive the optical fiber (20) therein.

## Description

### BACKGROUND OF THE INVENTION

The proper healing of bone and tissue depends on multiple factors. Some of these factors include the age and biology of a patient, type and severity of the patient's injuries, and infections. Healing can be monitored through radiography, blood sample analysis and the like. However, these current monitoring solutions are not suitable to predict either bone healing failures or normal bone formation. As such, complications, such as nonunions and surgical site infections, are often recognized too late in clinical practice.

Nonunions are a serious complication that can result in psuedarthrosis or poor bone alignment, which may require surgical intervention. Such nonunions may be caused by bone migration during the healing process, poor blood supply and infection, to name a few.

Surgical site infections (SSI) can occur in the wound created by an invasive surgical procedure and are a significant cause of healthcare-associated infections. Such infections are a general health risk to the patient and can lead to non-unions in bone healing, particularly when left untreated. Further information on the risks and complications of SSI's can be found in National Collaborating Centre for Women's and Children's Health, Surgical Site Infection Prevention and Treatment of Surgical Site Infection Clinical Guideline (2008).

Adequate and early prediction of nonunions and SSI's, along with other clinical problems, can help healthcare professionals implement early intervention to combat their occurrences and ill effects. However, current monitoring practices and techniques are inadequate. Bone healing progress is often monitored using radiography and evaluation of functional outcomes, such as pain. Infections are currently monitored by blood sampling and other techniques. However, all of these techniques have significant predictive limitations. Radiography is a non-continuous technique that cannot predict load bearing properties of the fracture site and is potentially hazardous especially if overused. Blood sampling and analysis is also non-continuous and valuable time for fighting an infection is often lost between blood draws. Moreover, other than their predictive limitations, radiography, blood analysis, and other clinical diagnostic measures often require physically limited patients to make burdensome trips to a healthcare facility just to receive such testing.

### BRIEF SUMMARY OF THE INVENTION

The present disclosure describes devices, systems, and methods for continuously monitoring conditions involved in the bone healing process to facilitate early recognition of complications so that early intervention can be realized. More particularly, the present disclosure describes a biocompatible optical fiber that is placed along an implant and/or fracture/osteotomy site to locally and continuously monitor site conditions such as fracture deflection, temperature, pressure, strain, pH, and other molecular characteristics. Such probe can be intraoperatively placed in an open manner or through an arthroscope or needle. Patches, glue, implant channels, sutures and other means of fixation may be used to hold the fiber in place during the healing process. Once healing and monitoring is complete, the probe can be extracted non-invasively. Features, such as an elastic sleeve, may help keep the probe in position during the healing process and facilitate extraction. Data gathered by the bone probe can be stored on a memory chip and sent to a display, mobile device, or healthcare provider for continuous monitoring.

In one aspect of the present disclosure, a bone healing monitoring system includes a probe configured to measure at least one physical condition and has an optical fiber and a control unit attached to the optical fiber. The system also includes and an implantable device that has a channel extending along at least a portion of the implantable device and is configured to receive the optical fiber therein.

Additionally, the control unit may include a laser and a detector. The optical fiber may also include at least one Bragg mirror sensor configured to return a wavelength of light to the detector indicative of the physical condition. The at least one physical condition may be one of deflection, pressure, strain, temperature and pH. The optical fiber may include a plurality of Bragg mirror sensors distributed along the length of the fiber each being tuned to a different bandwidth of light for returning a multiplexed signal to the detector. Each Bragg mirror sensor may comprise a section of the optical fiber defined by intervals of varying refractive index.

Continuing with this aspect, the laser may be a semiconductor laser. The control unit may include a processor and memory. The processor and memory may comprise a three-dimensional semiconductor package.

Also, the implantable device may be one of an elastic sleeve, joint prosthesis, intramedullary nail, bone plate, and external fixator. The surgically implantable device may be implantable by one of open surgery, arthroscopic surgery, and percutaneous needle insertion. The optical fiber may be removable from the channel once received therein. The channel may be formed along an outer surface of the implantable device.

In another aspect of the present disclosure, a bone healing monitoring system includes an implantable device having a length defined between a first and second end thereof, and a probe configured to measure at least one physical condition and having an optical fiber and a control unit attached to the optical fiber. The optical fiber is removably attachable to the implantable device between the first and second ends.

Additionally, the optical fiber may be removably attachable to the implantable device by an adhesive. The implantable device may include a channel extending into the implantable device between the first and second ends. The channel may be configured to receive the optical fiber. The implantable device may include a channel extending into the implantable device and may extend along an outer surface or an inner surface between the first and second ends. The channel may be configured to receive the optical fiber.

In a further aspect of the present disclosure, a bone healing monitoring system may include a probe configured to measure at least one physical condition and may have an optical fiber and a control unit attached to the optical fiber. The system also includes an intramedullary nail having interior and exterior surfaces defining a sidewall therebetween and extending along a length defined between first and second ends thereof. The intramedullary nail includes a channel that one of extends through the sidewall, along the outer surfaces, and along the inner surface. The channel is configured to removably receive the optical fiber therein.

In yet another aspect of the present disclosure, a bone healing monitoring system includes a probe configured to measure at least one physical condition and includes an optical fiber and a control unit attached to the optical fiber. The system also includes a bone plate having interior and exterior surfaces defining a sidewall therebetween and extending along a length defined between first and second ends thereof. The bone plate has a channel that one of extends through the sidewall, along the outer surface, and along an edge defined by an outer periphery of the sidewall. The channel is configured to removably receive the optical fiber therein.

In an even further aspect of the present disclosure, a bone healing monitoring system includes a probe configured to measure at least one physical condition and includes an optical fiber and a control unit attached to the optical fiber. The system also includes an external fixator having rod, a plurality of pins, and a channel extending through one of the rod and pins, the channel is configured to removably receive the optical fiber therein.

In a still further aspect of the present disclosure a method for monitoring bone healing of a fracture includes implanting a probe adjacent to a bone fracture of a patient, The probe is configured to measure at least one physical condition and has an optical fiber and control unit attached to the optical fiber. The method also include measuring the at least one physical condition adjacent to the bone fracture and removing the probe from the patient.

Additionally, implanting the probe may include intraoperatively placing the probe adjacent to the fracture through an arthroscope or needle. Also, implanting the probe may include attaching a first portion of the optical fiber having a first Bragg mirror sensor to a first bone segment and a second portion of the optical fiber having a second Bragg mirror sensor to a second bone segment. The first and second bone segments may be opposite each other across the bone fracture. Implanting the probe may also include inserting at least a portion of the optical fiber into the fracture between a first and second opposed bone fragments to be joined.

Continuing with this aspect, the method removing the probe may include pulling the probe from a location external to the patient. Also, the method may include projecting light through the optical fiber from a light source in the control unit and detecting reflected light by a detector in the control unit. Measuring may include determining a wavelength of the reflected light and converting the wavelength to a unit of measure indicative of the at least one physical condition. The at least one physical condition may be one of deflection, pressure, strain, temperature, and pH.

Also, implanting may be performed intraoperatively and measuring and removing may be performed postoperatively. The method may also include attaching the optical fiber to an implant, and implanting the probe may include implanting the implant along with the optical fiber attached thereto. The implant may be one of a bone plate and IM nail. Also, implanting the probe may include inserting the optical fiber into a channel disposed in an implant. The implant may be implanted prior to inserting the optical fiber therein.

In another aspect of the present disclosure, a method for monitoring bone healing of a fracture includes implanting an implant adjacent a fracture of a patient. The implant includes a probe attached thereto. The probe has an optical fiber and a controller coupled to the optical fiber. The method also includes measuring at least one physical condition with the probe and removing the probe from the implant and the patient while the implant remains implanted.

Additionally, the implant may be one of an external fixator, intramedullary nail, and bone plate. Also, implanting may be performed intraoperatively and measuring and removing may be performed postoperatively.

### BRIEF DESCRIPTION OF THE DRAWINGS

The features, aspects, and advantages of the present invention will become better understood with regard to the following description, appended claims, and accompanying drawings in which:
FIG. 1 shows a bone healing probe of the present disclosure including an optical fiber and control unit.
FIG. 2 is a diagram of the control unit of FIG. 1.
FIGS. 3A and 3B show an example methodology of the bone probe of FIG. 1.
FIG. 4A shows an example methodology of the bone probe of FIG. 1 in connection with of an intramedullary nail.
FIG. 4B is a cross-sectional view taken at line A-A of FIG. 4A.
FIG. 5A shows an example methodology of the bone probe of FIG. 1 in connection with a bone plate.
FIG. 5B is cross sectional view taken along line B-B of FIG. 5A.
FIG. 6A shows an example methodology of the bone probe of FIG. 1 in connection with an external fixator.
FIG. 6B is a partial view of the bone probe of FIG. 1 routed through a rod of the external fixator of FIG. 6A.
FIG. 7 shows an example methodology of the bone probe of FIG. 1 in the context of range of motion training.

### DETAILED DESCRIPTION

When referring to specific directions in the following discussion of certain implantable devices, it should be understood that such directions are described with regard to the implantable device's orientation and position during exemplary application to the human body. Thus, as used herein, the term "proximal" means close to the heart and the term "distal" means more distant from the heart. The term "inferior" means toward the feet and the term "superior" means toward the head. The term "anterior" means toward the front of the body or the face and the term "posterior" means toward the back of the body. The term "medial" means toward the midline of the body and the term "lateral" means away from the midline of the body. Also, as used herein, the terms "about," "generally" and "substantially" are intended to mean that deviations from absolute are included within the scope of the term so modified.

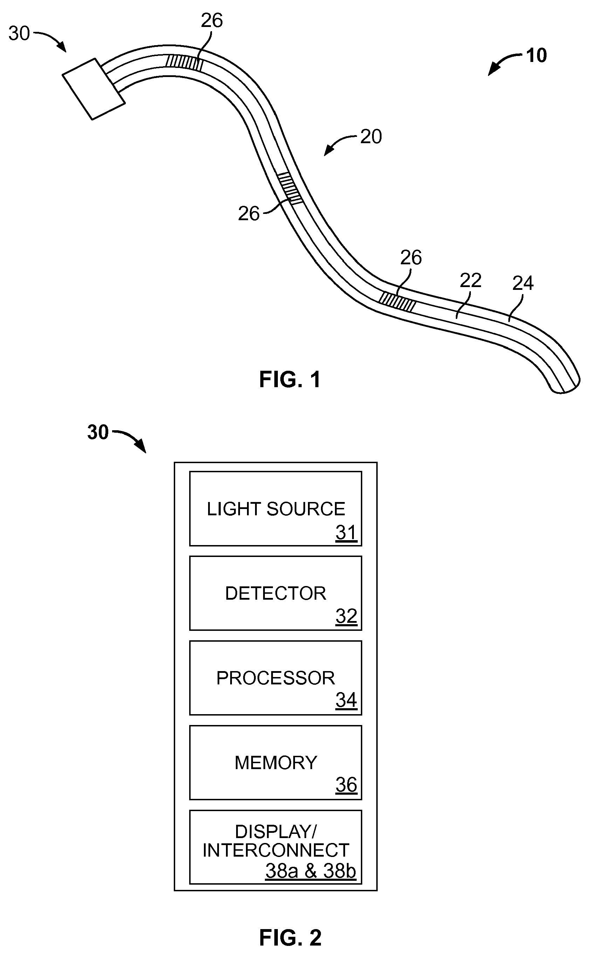

FIG. 1 depicts a bone healing probe system 10. Probe 10 generally includes an optical fiber 20 and a control unit 30. In some embodiments, multiple optical fibers 20 may be provided for a single control unit 30. Probe 20 is capable of being inserted into a human or animal body and placed at a bone fracture site where measurements are taken of one or more physical conditions. This information can be displayed at control unit 30, transmitted to a mobile device, and/or transmitted to the healthcare provider who can further evaluate the measurements to determine whether a failure mode in the healing process is indicated.

Optical fiber 20 includes a core 22 and cladding 24 surrounding core 20. Core 20 and cladding 24 can be dimensioned and made from any materials, such as SiO₂ and doped variants thereof, so that the result is total internal reflection of light within core 22. As shown, core 22 can include one or more Bragg mirror sensors 26 where each sensor 26 occupies a predefined length or section of core 22 and along which the refractive index of core 22 varies. In other words, each Bragg mirror sensor 26 comprises a section of optical fiber 20 defined by intervals of varying refractive index. As light is projected down optical fiber 20, each Bragg mirror sensor 26 reflects a wavelength of light toward controller 30, which is indicative of a physical condition sensed by sensors 26.

Each Bragg mirror sensor 26 along the length of optical fiber 20 can be configured to measure a different physical condition/parameter. Such physical conditions can include, but are not limited to, temperature, pressure, deflection of optical fiber 20, strain, pH levels, and gas concentration levels, such as O₂ and CO₂. The measurements of temperature, pH, and relative levels of O₂ and CO₂ at the fracture site can be used to indicate and predict bacterial infections. Pressure, deflection, strain and other mechanical conditions can be used to predict nonunion of bone fragments surrounding the fracture. Where optical fiber 20 includes a plurality of sensors 26, each sensor 26 can be tuned to operate within a predefined bandwidth of light so that a multiplexed signal is sent back to controller 30 where it can be demuxed by a processor in controller 30.

FIG. 2 illustrates an exemplary control unit 30. Control unit 30 includes a light source 31, detector 32, processor 34, memory 36, and display/interconnect 38. Control unit 30 can include each of these components in a single, self-contained unit attachable to optical fiber 20. However, in some embodiments, these components can be separated into multiple units that are attachable to one another via a physical or wireless interface.

Light source 31 is a laser, which is preferably supplied by a unit small enough to be attached to a human body, such as a semiconductor laser and the like. However, other larger units are contemplated.

Detector 32 is also preferably small enough to be attached to a human body, such as a photodiode and the like. Although, other larger units are contemplated.

Light signals detected by detector 32 are processed by processor 34, which may include converting detected wavelengths to practical units of measure, such as those units that describe the physical conditions mentioned above. Processor 34 may perform such processing based on instructions stored on memory 36, which may be flash memory or the like. The output from processor 34 may be stored on memory 36 in a continuous fashion. Thus, physical conditions can be continuously measured and stored. In one embodiment, memory 36 and processor 34 can be provided in a single 3D integrated circuit package in order to minimize the footprint occupied by memory 36 and processor 34 so that memory 36 and processor 34 can also be attached to a human body.

Controller 30 also includes display or interconnection 38a. In one embodiment, a small display 38a, such as an LED display, can be integrated into controller 30 for attachment to a human body along with the other components mentioned above. Such display may communicate with processor 34 and display physical conditions measured at the fracture site. For example, display 38a may continuously display the temperature at the fracture site. A patient can be instructed to notify their healthcare professional when the temperature reaches a certain threshold, which may be indicative of an SSI. Additionally, the display allows the patient to be intimately involved in the healing process by displaying information that allows the patient to track his or her progress. In one embodiment, the processor may display comparisons and/or graphs indicating the patient's progress. This can be highly motivating to the patient and can help the patient understand what activities, such as physical therapy, are most beneficial.

In another embodiment, controller 30 may include an interconnection 38b in lieu of or in conjunction with display 38a. Interconnection 38b may include physical hardware that allows controller 30 to be physically coupled to an external unit that can extract data stored on memory 36.

Alternatively, interconnection 38b can include circuitry or other hardware that provides a wireless connection with an external unit. For example, controller 30 can include Bluetooth® or Wi-Fi components. Such components can be integrated into the processor/memory package as a system on chip (SOC). Data can then be transferred from controller 30, wirelessly, to a mobile device, such as a smartphone or tablet. The mobile device can include an application that provides further processing and interactivity with the user. In addition, the application can serve as a link between probe 10 and healthcare provider where the mobile device application transmits information gathered by probe 10 to the healthcare provider for an up to the minute evaluation.

Controller 30 can include other components. For example, in the above description, Bragg mirror sensors 26 are utilized to sense physical conditions. However, in an alternative embodiment, rather than Bragg mirrors, optical time domain reflectometry (OTDR) and/or optical frequency domain reflectometry (OFDR) can be utilized to measure physical conditions at the fracture site. As such, controller 30 can contain additional components, such as a pulse generator, to facilitate such techniques.

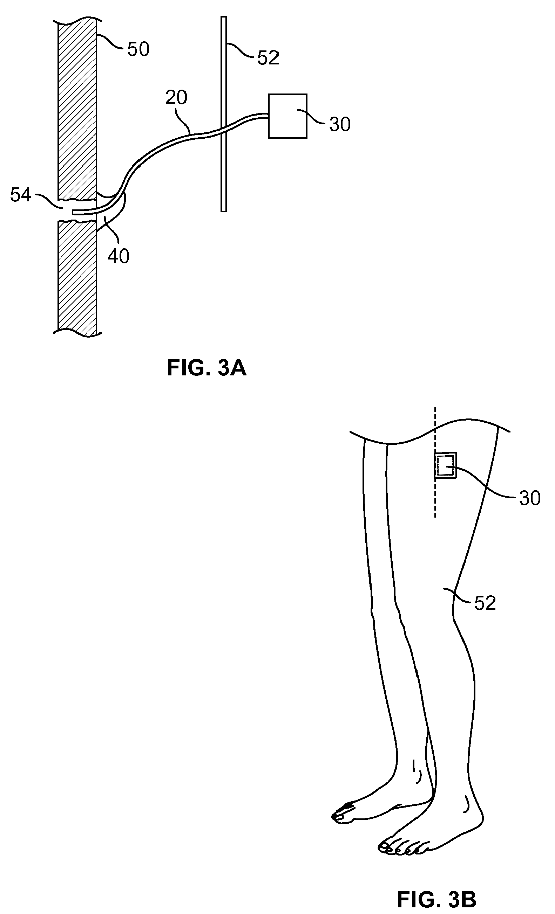

Exemplary applications of probe 10 to the human body for monitoring fracture healing are now described. FIGs. 3A and 3B depict an embodiment of a method of using probe 10 to measure physical conditions at a fracture site. In this embodiment, an elastic sleeve 40 may be provided to help guide, stabilize and position probe 10 in the desired location and may also help with removal of optical fiber 20 once healing is complete. Sleeve 40 includes a channel for receipt of optical fiber 20 and may include additional features for attachment to bone.

In the method, probe 10 is delivered to the fracture site by open surgery, an arthroscope, or a percutaneously inserted needle. Optical fiber 20 may be partially inserted into the fracture space between the bone fragments to be joined. As the fracture heals, fiber 20 can be repositioned, if needed, so that it does not interfere with the healing process.

Sleeve 40 is attached to bone 50 via an adhesive or to soft tissue via a resorbable suture. As shown, sleeve 40 holds optical fiber 20 in position so that the end of the optical fiber 20 extends into a fracture space 54. The end of optical fiber 20 may include a sensor 26 to measure conditions within fracture space 54. Fiber 20 may also include various sensors 26 along its length to measure conditions farther away from fracture space 54 in order to obtain a more complete picture of fracture site conditions.

In one embodiment, fiber 20 itself may be attached to bone 50. In such an embodiment, one sensor 26 may be attached to a first bone fragment, and another sensor 26 may be attached to a second bone fragment disposed opposite the first bone fragment across fracture space 54. Alternatively or in addition to any one of the previously described embodiments, a single sensor 26 within fiber 20 can be placed so that sensor 26 spans across fracture 54.

With optical fiber 20 in place and at least partially extending from the patient's skin 52, controller 30 is attached to fiber 20, if not already attached, and controller 30 is attached to outer skin 52, which may be achieved by an adhesive patch, suture, tape or the like (see FIG. 3B).

Controller 30 is activated and light source 31 directs light down fiber 20. Reflected wavelengths or backscatter is detected by detector 32 and signals are sent directly or indirectly (such as through an amplifier) from detector 32 to processor 34, which converts the detected wavelengths into units of the desired physical condition for measurement. Outputs from processor 34 are stored on memory 36. Fiber 20 remains in place during the healing process and continuously monitors fracture site conditions.

Real time conditions, such as temperature, pressure, pH, deflection, and the like are sent to display 38a within controller 30 and attached to patient's skin 52. Alternatively, conditions are uplinked periodically to a mobile device from controller 30, which backs-up the data and can further process the data via a specialized application for enhanced user functionality over that of display 38a. User may also forward data to a healthcare professional and/or the healthcare professional can retrieve data from the mobile device on command when connected to a wireless network. Over time, the mobile device application can be modified and can learn from data collected and assist in warning users that certain conditions, such as those indicating an SSI or nonunion, exist that warrant notifying a healthcare professional.

Once healing is completed, optical fiber 20 is noninvasively removed from the fracture site and patient. This may be performed by detaching controller 30 from the patient and pulling optical fiber 20 through skin 30. Alternatively, optical fiber 20 can be removed arthroscopically. While fiber 20 is preferably removed from the patient, it is contemplated that controller 30 is detached from the patient while fiber 20 remains inside the patient.

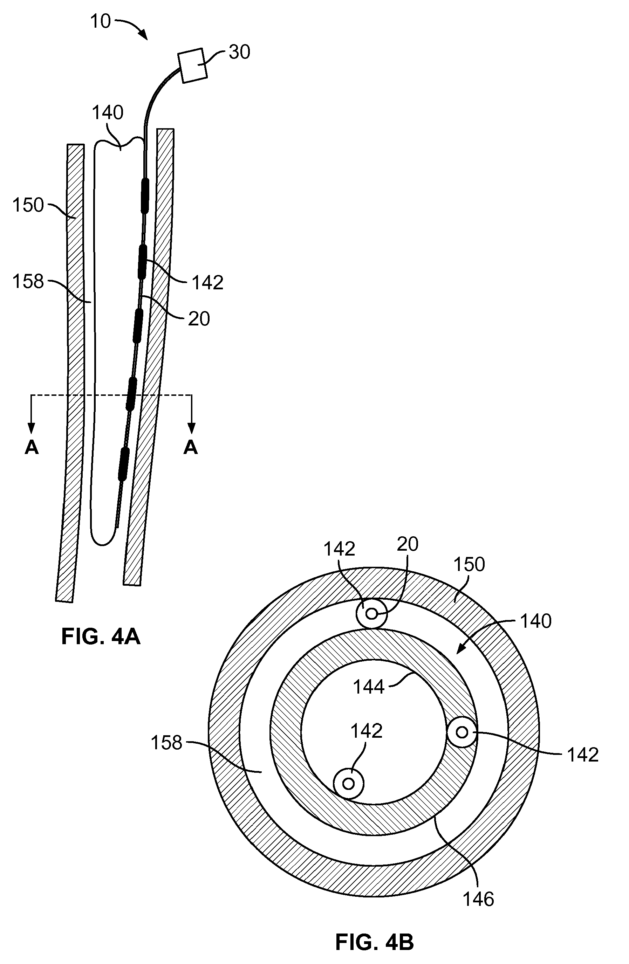

FIGs. 4A and 4B illustrate a method of implementing probe 10, in conjunction with an intramedullary (IM) nail 140. IM nail 140 includes first and second ends and a length defined therebetween. As shown in the cross-sectional view of FIG. 4B, IM nail 140 also includes interior and exterior surfaces 144, 146 and a sidewall defined therebetween.

In some embodiments, IM nail 140 may be specifically designed to accommodate probe 110, and in particular, optical fiber 20. In such a design, IM nail 140 may include one or more channels 142 longitudinally extending along IM nail 140. As shown, a channel 142 can be formed in the sidewall, on interior surface 144, and/or on exterior surface 146. Channels 142 on interior and exterior surfaces 144 and 146 may be formed by a sleeve attached one of these surfaces that either extends continuously along the length of nail 140 or extends in intervals along the length of nail 140 (as depicted in FIG. 4A) such that transverse openings are formed to expose a portion of optical fiber 20 when disposed therein. Each channel 142 is configured to receive and hold optical fiber 20 during the healing process, while allowing optical fiber 20 to be removed when desired. In other embodiments, probe 10 may be attached to a standard or non-specialized IM nail by adhesive or other reversible means.

In a method of use, optical fiber 20 is attached to IM nail 140 by sliding fiber 20 into a respective channel 142 in IM nail 140. This may be done during the manufacturing process and delivered to the operating room in an assembled configuration, or it may be performed in the operating room. Alternatively, adhesive may be attached to IM nail 140 and optical fiber 20 attached to the adhesive. One or more optical fibers 20 may be attached to IM nail 20 as described.

Nail 140, along with optical fiber 20, is inserted into an IM canal 158 of a bone 150 such that optical fiber 20 extends from nail 140 to a location outside of the patient. Alternatively, nail 140 is inserted into IM canal 158 and then optical fiber 20 is guided into channel 142.

With optical fiber 20 in place and at least partially extending from the patient's skin, controller 30 is attached to fiber 20, if not already attached, and controller 30 is attached to the patient's outer skin, which may be performed by an adhesive patch, tape or the like.

Real time conditions, such as temperature, pressure, pH, deflection, and the like are sent to display 38a within controller 30 and attached to patient's skin 52. Alternatively, conditions are uplinked periodically to a mobile device from controller 30, which backs-up the data and can further process the data via a specialized application for enhanced user functionality over that of display 38a. User may also forward data to a healthcare professional and/or the healthcare professional can retrieve data from the mobile device on command when connected to a wireless network. Over time, the mobile device application can be modified and can learn from data collected and assist in warning users that certain conditions, such as those indicating an SSI or nonunion, exist that warrant notifying a healthcare professional. While conditions may be measured continuously without pause, conditions can also be measured at intervals selected to conserve energy of the controller's power source while still providing up to the minute information.

Once healing is completed, optical fiber 20 is noninvasively removed from IM nail 140 and the patient. This may be performed by detaching controller 30 from the patient and pulling optical fiber 20 through the skin.

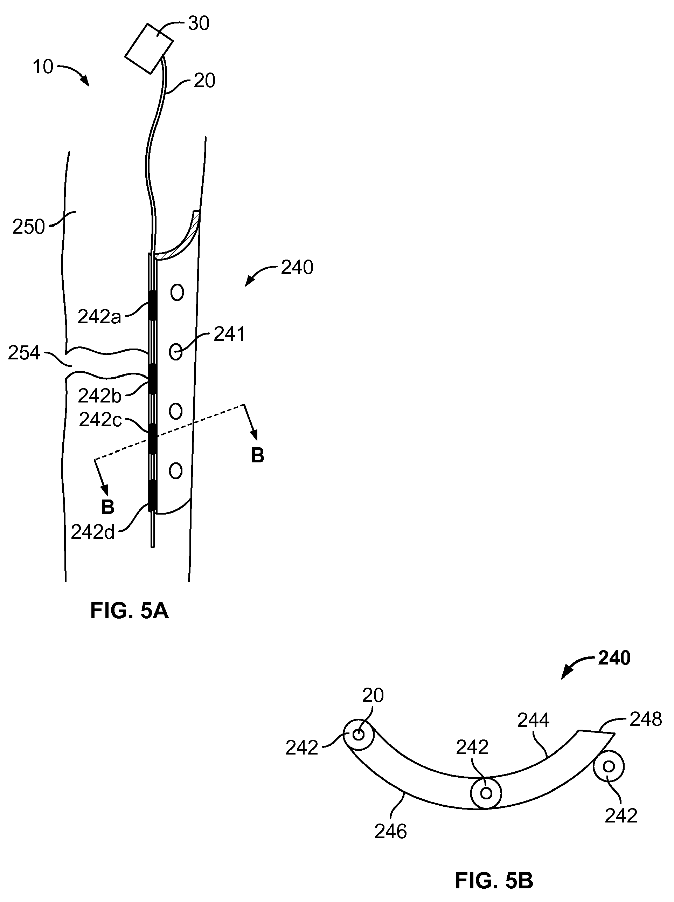

FIGs. 5A and 5B illustrate a method of implementing probe 10 in conjunction with a bone plate 240. Bone plate 240 includes first and second ends and a length defined therebetween. As shown in the cross-sectional view of FIG. 5B, the bone plate also includes interior and exterior surfaces 244, 246 and a sidewall defined therebetween. The sidewall terminates at a periphery which defines an edge 248. Bone plate 240 may also include a plurality of transverse through-holes 241 extending through the sidewall for receipt of a bone fastener.

In some embodiments, bone plate 240 may be specifically designed to accommodate probe 10, and in particular, optical fiber 20. In such a design, bone plate 240 may include one or more channels 242 longitudinally extending along plate 240. As shown, channels 242 can be formed in the sidewall, on exterior surface 246, and/or on edge 248 of the sidewall. Channels 242 on exterior surface 246 or edge 248 may generally be formed by a sleeve attached exterior surface 246 or edge 248 that either extends continuously along the length of plate 240 or extends in intervals (as shown in FIG. 5A by intervals 242a-d) along the length of bone plate 240 such that transverse openings are formed to expose a portion of optical fiber 20 when disposed therein. Channel 242 that extends through the sidewall in a longitudinal direction may be located in a portion of the bone plate that does not intersect transverse through-holes 241. Each channel 242 is configured to receive and hold optical fiber 20 during the healing process, while allowing optical fiber 20 to be removed when desired. In other embodiments, probe 10 may be attached to a standard or non-specialized bone plate by adhesive or other reversible means.

In a method of use, optical fiber 20 is attached to bone plate 240 by sliding optical fiber 20 into a respective channel 242 in plate 240. This may be done during the manufacturing process and delivered to the operating room in an assembled configuration, or it may be performed in the operating room. Alternatively, adhesive may be attached to bone plate 240 and optical fiber 20 attached to the adhesive. One or more optical fibers 20 may be attached to bone plate 240 as described.

With the fracture properly aligned, bone plate 240 is placed across fracture 254 and secured to opposing segments of bone 250 via bone fasteners. Optical fiber 254 may be inserted into channels 242 and connected to bone plate 240 once bone plate 240 is secure. Alternatively, optical fiber 20 may be inserted into respective channels 242 prior to fixation of bone plate 240 to bone 250.

With optical fiber 20 in place and at least partially extending from the patient's skin, controller 30 is attached to fiber 20, if not already attached, and controller 30 is attached to the patient's outer skin, which may be performed by an adhesive patch, tape or the like.

Real time conditions, such as temperature, pressure, pH, deflection, and the like are sent to display 38a within controller 30 and attached to patient's skin 52. Alternatively, conditions are uplinked periodically to a mobile device from controller 30, which backs-up the data and can further process the data via a specialized application for enhanced user functionality over that of display 38a. User may also forward data to a healthcare professional and/or the healthcare professional can retrieve data from the mobile device on command when connected to a wireless network. Over time, the mobile device application can be modified and can learn from data collected and assist in warning users that certain conditions, such as those indicating an SSI or nonunion, exist that warrant notifying a healthcare professional.

Once healing is completed, optical fiber 20 is noninvasively removed from bone plate 240 and the patient. This may be performed by detaching controller 30 from the patient and pulling optical fiber 20 through the skin.

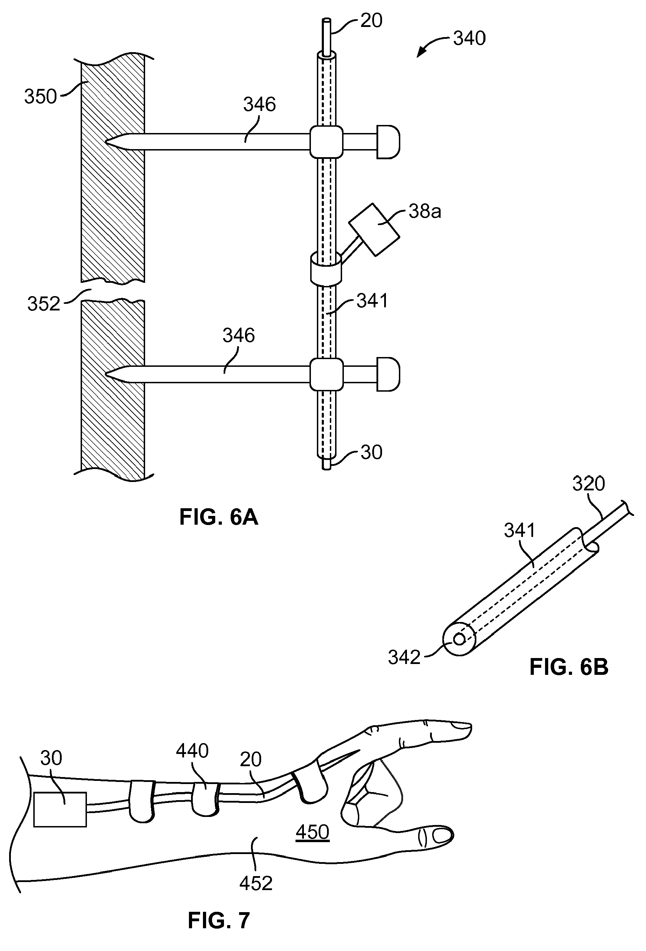

FIGs. 6A and 6B illustrate a method of implementing probe 10 in conjunction with an external fixator 340. External fixator 340 includes a rod 341 and pins 346 assembled into a construct where pins 346 adjustably extend from rod 342 in a transverse direction therefrom. Other embodiments of external fixator 340 may include fixation frames and/or a plurality of rods.

As shown in FIG. 6B, rod 342 includes a channel 342 extending along a length thereof for receipt of optical fiber 20. Such channel 342 extends through the entirety of rod 341 in one embodiment, and extends partially through rod 341 in another embodiment. In addition, pins 346 may also include such a channel for receipt of fiber 20. While probe 10 may not be implanted into the patient when optical fiber 20 is inserted into rod 341, such probe 10 is still useful to evaluate the stiffness of the fixator construct 340, any adjustments that could be made to optimize the performance of fixator 340, and conditions indicating a failure mode of healing. This could be done by using probe 10 to measure certain physical conditions felt by rod 342, such as pressure, strain, and deflection.

In a method of use, pins 346 are inserted into bone fragments disposed opposite each other across the fracture space 352. Pins 346 are coupled to rod 342 and adjusted in order to place the bone fragments into a desired relative position. Thereafter, optical fiber 20 may be inserted into channel 342 extending through rod 341. Controller 30 is connected to one end of optical fiber 20 and connected to an end of rod 341. In one embodiment, display 38a, which may be physical separated from controller unit 30 but in communication therewith, may be attached to rod 342 (as shown in FIG. 6A) to display real time conditions.

Alternatively, or in addition to optical fiber 20 in rod 341, fiber 20 can be inserted into each of pins 346. This may be done such that an end of fiber 20, which may include a sensor 26 for measuring temperature or some other condition, is placed within a tip of pins 346, which themselves are inserted into bone 350. Controller 30 can be attached to each fiber extending into pins 346 and attached to rod 341.

Real time conditions, such as temperature, pressure, pH, deflection, and the like are sent to display 38a within controller 30. Alternatively, conditions are uplinked periodically to a mobile device from controller 30, which backs-up the data and can further process the data via a specialized application for enhanced user functionality over that of display 38a. User may also forward data to a healthcare professional and/or the healthcare professional can retrieve data from the mobile device on command when connected to a wireless network. Over time, the mobile device application can be modified and can learn from data collected and assist in warning users that certain conditions, such as those indicating an SSI or nonunion, exist that warrant notifying a healthcare professional.

Where more than one optical fiber 20 is used, such as in each of pins 346, measurements obtained from each fiber 20 can be processed by controller 30. These measurements may be compared by a comparator, which can determine relative position or conditions at each bone fragment to be joined by the healing process. Once healing is completed, optical fiber 20 can be removed from the patient along with external fixator 340.

FIG. 7 illustrates a method of implementing probe 10 in the context of rehabilitation and range of motion training/evaluation. Evaluating range of motion of a joint (ROM) after an injury and/or surgical procedure involving the joint may be important in evaluating recovery progress and strategies for rehabilitation. As shown, probe 10 can be attached to a patient's outer skin 450 and across the joint 452 to be evaluated. In this example, a wrist. Other examples not shown, are knee, ankle, shoulder, elbow, and finger joints. Controller 30 is also attached to patient's outer skin 450. Probe 10 can be worn during a physical therapy session or can be worn for more prolonged durations to obtain additional data.

Aside from helping patients recover from injuries or surgeries by evaluating ROM, probe 10 can be used to evaluate ROM of a healthy joint to better understand their kinematics and to evaluate forces and other external stimuli applied to the body in everyday life, which can help improve implant design.

Probe 10 and methods described herein provide many benefits and advantages. For example, patients are currently unaware of their healing progress and rely on their healthcare provider's interpretation of certain diagnostic tools, such as x-rays. Probe 10 and methods of using the probe allows the patient to be continuously aware of their healing progress, which may greatly motivate patients. In addition, healthcare providers can use the data and experience obtained from utilizing such probes and methods to identify optimum rehabilitation strategies. Additionally, optical fiber 20 is a passive device that does not transmit electrical or magnetic energy inside the body, which helps avoid foreign body reactions, and continuously monitors fracture site conditions so that the patient and/or healthcare provider can obtain up to the minute information, which can be used to predict failure modes in the healing process.

Although probe 10 has generally been described above as being applicable to predicting failure modes in the processes of bone fracture healing, it should be understood, that such probe has other clinical applications. Probe 10 can be used to monitor any wound, injury, or surgical site for proper healing and development of infections. In addition, probe 10 can be used in other orthopedic contexts not shown. For example, probe 10 can be utilized in conjunction with joint arthroplasty prostheses to evaluate proper joint alignment and fixation of the prosthesis. Other examples include monitoring of bone formation in distraction osteogenesis or relapse of osteotomies. Even further still, probe 10 can be used to determine or predict implant failure, for example, if bending exceeds a certain value. Such determination allows the healthcare professional to remove the implant before failure occurs. Regardless of the application, the principles described herein are applicable.

Although the invention herein has been described with reference to particular embodiments, it is to be understood that these embodiments are merely illustrative of the principles and applications of the present invention. It is therefore to be understood that numerous modifications may be made to the illustrative embodiments and that other arrangements may be devised without departing from the spirit and scope of the present invention as defined by the appended claims.

## Claims

1. A bone healing monitoring system, comprising:
a probe configured to measure at least one physical condition and having an optical fiber and a control unit attached to the optical fiber; and
an implantable device having a channel extending along at least a portion of the implantable device and being configured to receive the optical fiber therein.

2. The system of claim 1, wherein the control unit includes a laser and a detector.

3. The system of claim 2, wherein the optical fiber includes at least one Bragg mirror sensor configured to return a wavelength of light to the detector indicative of the physical condition.

4. The system of claim 3, wherein the at least one physical condition is one of deflection, pressure, strain, temperature and pH.

5. The system of claim 2, wherein the optical fiber includes a plurality of Bragg mirror sensors distributed along the length of the fiber each being tuned to a different bandwidth of light for returning a multiplexed signal to the detector.

6. The system of claim 5, wherein each Bragg mirror sensor comprises a section of the optical fiber defined by intervals of varying refractive index.

7. The system of claim 2, wherein the laser is a semiconductor laser.

8. The system of claim 7, wherein the control unit includes a processor and memory.

9. The system of claim 8, wherein the processor and memory comprise a three-dimensional semiconductor package.

10. The system of claim 1, wherein the implantable device is one of an elastic sleeve, joint prosthesis, intramedullary nail, bone plate, and external fixator.

11. The system of claim 1, wherein the surgically implantable device is implantable by one of open surgery, arthroscopic surgery, and percutaneous needle insertion.

12. The system of claim 1, wherein the optical fiber is removable from the channel once received therein.

13. The system of claim 1, wherein the channel is formed along an outer surface of the implantable device.

14. The system of claim 1, wherein the probe is removable from the channel while the implantable device remains implanted.

15. The system of claim 14, wherein the probe is removably attachable to the implantable device by an adhesive.
